# EUROPEAN PATENT APPLICATION

(11) **EP 1 759 707 A2**
(43) Date of publication of application: **07.03.2007**
(21) Application number: 06254357.4
(22) Date of filing: 18.08.2006
(51) Int. Cl.: A61K 38/20, A61K 39/44

(54) **Canine tumor treatment method, pharmaceutical formulation applied thereto and method of cryogenically preserving cells used therewith**

(30) Priority: 22.08.2005 JP 2005240172
(71) Applicant: Lymphotec Inc., Tokyo 112-0001 (JP)
(72) Inventor: Ishii, Hiroshi, Tokyo, 1670054 (JP); Bamba, Kenzo, Shimotuma-shi Ibaraki, 3040068 (JP); Iinuma, Akiko, Yokohama-shi Kanagawa (JP); Kuroiwa, Yasuyuki, Hitachinaka-shi Ibaraki, 3120062 (JP); Osumi, Kazuoki, Ami-machi Inashiki-gun Ibaraki, 3001152 (JP); Sekine, Teruaki, Koto-ku 1350043 (JP)
(74) Representative: Murphy, Colm Damien

(57) **Abstract**

A treatment for tumorous canines and a method of preparing, cultivating, and cryogenically preserving the pharmaceutical formulation used in the treatment. Canine lymphocytes are activated and expanded in the presence of a combination of anti-canine CD3 antibodies and interleukin-2 (IL-2) (preferably anti-canine CD3 antibodies and human interleukin-2 (human IL-2)) and administered to tumorous dogs. Following treatme-nt, the canine tumors were observed to be in regression while no serious side effects were noted.
Quality-of-life improvements were observed in the form of the animals' ability to walk increased distances and improved coat appearance.

## Description

### BACKGROUND OF THE INVENTION

The present invention relates to a method for treating canine tumor, a pharmaceutical formulation which uses canine lymphocytes to treat a tumorous canine, and a method of culturing and cryogenically preserving cells used for the treatment.

J.H. Jardine et al have reported that canine lymphocytes may be exlpanded using human interleukin 2 (hIL-2) (Veterinary Immunology, Volume 21, 1989, pp. 153-160). Moreover, D. W. Mitchell et al have reported that propagation may be conducted using PHA (Phytohemaglutinin) and hIL-2 (American Journal of Veterinary Research, Volume 52, 1991, pp. 1,132-1,136).

Furthermore, H. Itoh et al have reported that canine lymphocytes may be propagated with solid phase anti-canine CD3 antibodies and hIL-2 (Journal of Veterinary Medical Science, Volume 65, 2003, 329-333). In addition, Mizuno et al have reported that canine lymphocytes may be cultivated with PHA and IL-2 at a temperature of 38°C (Mizuno et al, Experimental Animal, Volume 45, 1996, 292).

Reports by J. H. Jardine et al and D. W. Mitchell et al, however, disclose that canine lymphocytes may be propagated with hIL-2 alone to produce LAK (Lymphokine Activated Killer) cells derived from large granular lymphocytes (LGL). A report by D. W. Mitchell discloses that canine peripheral lymphocytes have been cultivated with PHA and hIL-2, and that PHA activates T-cells via CD2 molecules on the surface of the T-cell while the anti-CD3 antibodies activate T-cells via CD3. Therefore, cells cultivated in this manner have a different activation mechanism and therefore exhibit a different T-cell population and function.

Moreover, H. Itoh et al confirmed that no adverse effects were observed by the infusion of activated lymphocytes obtained by cultivating canine lymphocytes with solid-phase anti-canine CD3 antibodies and hIL-2, but they did not confirm that there was anti-tumor activity in vivo. Furthermore, Mizuno et al propagated canine lymphocytes with PHA and IL-2 at a temperature of 38°C, but did not confirm any anti-tumor effect in vivo. The Mizuno and Itoh's methods for activating lymphocytes are different from the CD3 activation method specified by the present invention in that these methods do not provide effective treatment for a canine tumor.

In regard to therapies applied to tumorous canines, as of the present there have been no anti-cancer drugs developed for canines. As a result, anti-cancer drugs developed for human beings have been administered to tumorous canines at dosages calculated according to body surface area. The safety of the treatment and its anti-tumor effects, however, have not been sufficiently confirmed. Furthermore, although many clinical trials have made use of activated lymphocytes for human cancer therapies, there have been no confirmed reports regarding the safety of these therapies, improvements in the patients' quality of life, nor the anti-tumor effects of activated lymphocytes on tumorous canines.

### SUMMARY OF THE INVENTION

The invention was construed through a process in which inventors activated and propagated canine peripheral blood lymphocytes in the presence of a combination of solid-phase anti-canine CD3 antibodies and interleukin-2 (IL-2) with a preferred combination being anti-canine CD3 antibodies and recombinant human interleukin-2 (hIL-2). These were administered to tumorous canines after which beneficial results were observed in the form improvements in a quality-of-life index and anti-tumor effect.

The Claim 1 invention relates to a method of treating canine tumor wherein the peripheral blood lymphocytes of a canine are propagated and activated using a combination of solid-phase anti-canine CD3 antibodies and interleukin-2 (IL-2) after which the resulting lymphocytes are administered to a tumorous canine.
Moreover, the Claim 2 invention relates to a method for treating canine tumor according to Claim 1 wherein the interleukin-2 (IL-2) is a human interleukin-2 (hIL-2).

The Claim 3 invention relates to the method for treating canine tumor according to Claim 1 wherein the temperature under which canine peripheral blood lymphocytes are propagated and activated lies within a range of from 37°C to 41 °C.
Moreover, the Claim 4 invention relates to a method for treating canine tumor according to Claim 1 wherein the temperature under which canine peripheral blood lymphocytes are propagated and activated lies within a range of from 38°C to 40°C.

The Claim 5 invention relates to a pharmaceutical formulation for treating a canine tumor wherein the formulation is obtained from the propagation and activation of canine peripheral blood lymphocytes in the presence of a combination of solid-phase anti-canine CD3 antibodies and interleukin-2 (IL-2).
Moreover, the Claim 6 invention relates to a pharmaceutical formulation for treating a canine tumor according to Claim 5 wherein the interleukin-2 (IL-2) is a human interleukin-2 (hIL-2).

The Claim 7 invention relates to a pharmaceutical formulation for treating a canine tumor according to Claim 5 wherein the temperature under which the canine peripheral blood lymphocytes are propagated and activated lies within a range of from 37°C to 41°C.
Furthermore, the Claim 8 invention relates to a formulation for treating a canine tumor according to Claim 5 wherein the propagation and activation temperature of the canine peripheral blood lymphocytes lies within a range of from 38°C to 40.°C.

The Claim 9 invention relates to a method of preparing cryogenically preserved cells for treating a canine tumor wherein peripheral blood lymphocytes of a canine are propagated and activated in the presence of a combination of solid-phase anti-canine CD3 antibodies and interleukin-2 (IL-2) after which the resulting lymphocytes are cryogenically preserved for subsequent thawing and preparation for use as a pharmaceutical formulation.

The Claim 10 invention relates to a method of cryogenically preserving cells for treating a canine tumor according to Claim 9 wherein the interleukin-2 (IL-2) is human interleukin-2 (hlL-2).

The Claim 11 invention relates to a method of cryogenically preserving cells for treating a canine tumor according to Claim 9 wherein the temperature under which the canine peripheral blood lymphocytes are propagated and activated in vitro lies within a range of from 37°C to 41°C. Moreover, the Claim 12 invention relates to a method of cryogenically preserving cells for treating a canine tumor according to Claim 9 wherein the temperature under which the canine peripheral blood lymphocytes are propagated and cultivated in vitro lies within a range of from 38°C to 40°C.

As noted above, the canine tumor treatment cells utilized in the treatment method, pharmaceutical formulation, and cryogenic preservation method specified by the invention are activated and propagated in the presence of a combination of solid-phase anti-canine CD3 antibodies and interleukin-2 (IL-2), preferably in the presence of a combination of anti-canine CD3 antibodies and human recombinant interleukin-2 (hlL-2). These cells provide a clearly recognizable tumor-reducing effect when administered to a tumorous canine as activated lymphocytes. No serious side effects were observed after administration of the formulation, an improvement in quality-of-life was noted in the form of the ability of the treated animals to walk longer distances, and an improvement in the animals' health was noted in the form of a healthier appearing coat.

### BRIEF DESCRIPTION OF THE DRAWINGS

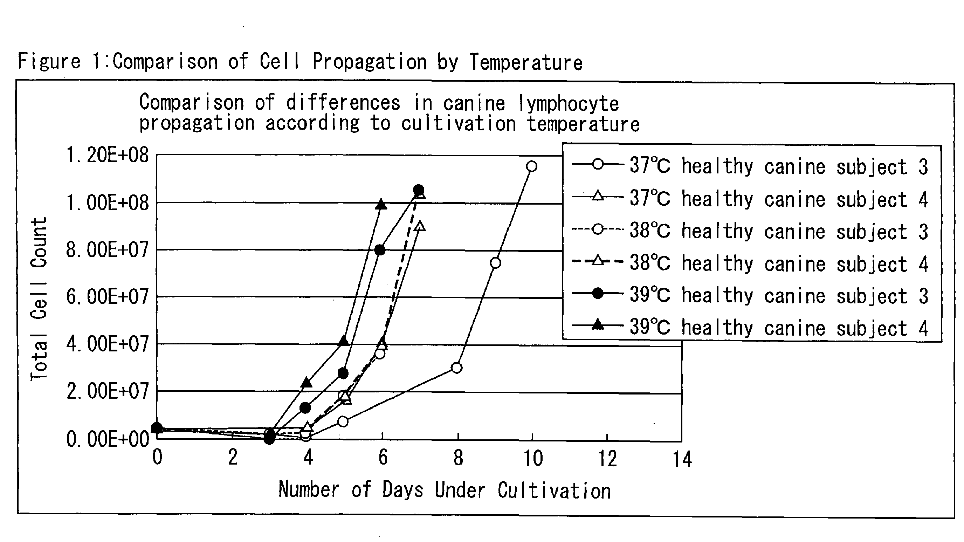

Fig. 1 is a line drawing comparing cell propagation levels at different cultivation temperatures.

### DETAILED DESCRIPTION OF THE INVENTION

A detailed description of the invention is provided by the following preferred embodiments.

### [First Embodiment]

Lymphocytes collected separately from two healthy test canines were cultivated twice each according to the following procedure.

### Procedure 1: Preparation of Cell Cultivation Flasks

Five milliliters (5ml) of an anti-canine CD3 antibody (Serotec Ltd., mouse anti-canine CD3) solution diluted to 5µg/ml with a physiological saline solution (Hikari Pharmaceuticals, Ltd.) were placed in a 75cm² culture flask (Sumitomo Bakelite Co., Ltd.) to a level which covered the bottom of the flask.

The flasks were maintained at 4°C (±2°C) overnight after which the anti-canine CD3 antibody solution in the flask was discarded. Twenty milliliters (20ml) of the physiological saline solution was poured into the flasks after which it was sealed with a cap and thoroughly agitated. The caps were then removed and the solution discarded. This operation was repeated after which the liquid remaining in the flask and cap was removed and the flasks placed in a refrigerator until further use.

### Procedure 2: Blood Collection

Five milliliters (5ml) of peripheral blood were separately collected and heparinized from healthy canine test subjects 1 and 2 weighing 2kg and 22kg respectively..

### Procedure 3: Separation of Peripheral Blood Mononuclear Cells

The peripheral blood was aseptically placed in a 15ml centrifuge tube (Greiner Bio-One Co., Ltd; No.188271) in a clean bench (Showa Science Co., Ltd; No.S-1100PRV) and separated for 10 minutes in a centrifugal separator (Kokusan Co., Ltd. No.H-700FR) at 1,800 rpm at 24 °C.

The centrifugation process separated and precipitated the erythrocytes, leukocytes, and lymphocytes from the supernatant plasma. The plasma was then cryogenically preserved and put aside for future use. In order to separate the lymphocytes from the remaining blood cell layers, the layers were diluted with approximately twice their volume of washing solution [500ml of RPMI-1640 cultivating medium (Nikken Bio Medical Laboratories Inc.), 3ml of 1 N hydrochloric acid (Wako Pure Chemical Industries, Ltd.), and 10ml of 1 M HEPES solution (Sigma-Aldrich Co., No.H0887)] and were added in a 50ml centrifuge tube (Greiner Co., Ltd., No. 227216).
Three milliliters (3ml) of Lymphosepar 1 (Immuno-Biological Laboratories, Ltd., No.23010) was subsequently added to a 15ml centrifuge tube using a 5ml pipette (Corning Japan K.K., No.4487). Ten milliliters (10ml) of the blood cells suspended in a washing solution were then gently layered onto the Lymphosepar 1 without disturbing the interface. The solution was then separated in a centrifuge at 1,600 rpm at 24°C for 30 minutes with the brake off.

After centrifugation, a fraction of the lymphocytes was taken from the interfacial layer and placed in a 50ml centrifuge tube (Greiner Bio-one Co., Ltd: No.227216) with a 5ml pipette, the washing solution was added to attain a volume ranging from 20 to 30ml, and the solution was mixed well. Ten microliters (10µl) of the solution was taken for counting the peripheral blood lymphocytes, and 500µl each of the solution was placed into each of three round bottom tubes (Beckton-Dickinson Japan Co., Ltd. No.352008) for analyzing cell surface antigens.

### Procedure 4: Counting the Collected Cells

The 10µl sample collected for the purpose of counting the number of peripheral blood mononuclear cells in Procedure 3 was mixed with 40µl of Turks solution (Muto Pure Chemicals Co., Ltd.) after which 10µl was placed=in an improved Neubeier Hematocytometer (Erma Inc.) for counting the cell number under a microscope. A total of 2.1×10⁷ and 1.1 ×10⁷ peripheral blood mononuclear cells were counted for the healthy canine subject No.1, and 6.4×10⁶ and 6.9×10⁶ were counted for the healthy canine subject No.2.

### Procedure 5: Analysis of Cell Surface Antigens

The samples collected for cell surface antigen analysis in Procedure 3 were separated in a centrifuge at 24°C at 1800 rpm for 5 minutes after which the supernatants were discarded. Into each of three tubes were respectively added (1) 5µl of control γ1/γ1 antibody (Becton, Dickinson and Company: 349526), (2) 5µl of a 1:1 mixture of FITC mouse anti-canine CD3 antibody (Serotec Co., Ltd.; No.MCA1774F) and PE mouse anti-canine B cell antibody (Serotec Co., Ltd. No.MCA178PE), and (3) 5µl of FITC rat anti-canine CD4/PE rat anti-canine CD8 antibody. These solutions where then reacted at 4°C for at least 10 minutes.

After reaction, 2ml of IsoFlow sheath solution (Becton, Dickinson and Co., No.C412006) containing 2% human albumin were added to each test tube and the contents of each mixed with a vortex mixer (Scientific Industry Co., No.4781487). The contents of the test tubes were then separated in a centrifuge at 24°C and 1,800 rpm for 5 minutes after which as much of the supernatant as possible was discarded. Analysis was conducted after adding 500µl of 2% human albumin IsoFlow sheath solution to the test tubes on the day, while 500µl of Cell Fix (Becton, Dickinson and Company: No.340181) was added to the tubes for the analysis on the next day. Analysis of cell surface antigens was performed with a FACS Calibur 4A (Becton, Dickinson and Company). The results are shown in Table 1.

**Table 1: Analysis of Cell Surface Antigens Before Cultivation**

| | Healthy Canine 1 | | Healthy Canine 2 | |
|---|---|---|---|---|
| | 1 | 2 | 1 | 2 |
| CD3 positive T cell | 67.1% | 70.5% | 35.7% | 40.3% |
| B cell positive cell | 9.3% | 14.5% | 13.3% | 19.5% |
| CD4 positive cell | 36.3% | 38.1% | 26.0% | 30.5% |
| CD8 positive cell | 26.3% | 25.0% | 21.2% | 22.5% |

### Procedure 6: Peripheral Blood Mononuclear Cell Cultivation

After sampling, the remaining peripheral blood mononuclear cells were separated in a centrifuge at 24°C at 1600 rpm for 10 minutes after which the supernatant was discarded by decanting and the cell pellets dispersed with a vortex mixer. The cells were suspended in 20ml of the cultivating medium [400ml of RPMI-1640+7s; (Nikken Bio Medical Laboratory Inc.), 4.5ml of 35,000IU/ml rIL-2 (Proleukin: Chiron Corp.), 45ml of FBS (JRH Biosciences, Inc.)] and transferred to a flask prepared as described in Procedure 1. The flask was then put into a large air jacket CO₂ incubator and incubated at a temperature of 37°C (±0.2°) in an environment having a CO₂ concentration of 5% (± 0.2%), and 95% (±5%) humidity.

### Procedure 7: Propagating and Expanding Cell Culture

The cells cultivated according to Procedure 6 were observed under a microscope, and 10 to 30ml of the same cultivating medium was added three to seven days after cultivation was initiated. The cultivating medium was added until the volume in the flask reached 50ml after which cultivation continued. The cells were transferred to a new flask, together with the cultivating medium, four to eight days after cultivation commenced. Fifty milliliters (50ml) of a fresh cultivating medium were added after which cultivation was allowed to continue. Six to ten days from the start of cultivation, 150ml of the cultivating medium, which contained hIL-2 175 IU/ml and 10% FBS, was added and cultivation allowed to continue for eight to sixteen days.

### Procedure 8: Inactivating the Autologous Serum

The blood serum collected in Procedure 3 was thermally inactivated in a water bath at 57°C for 50 minutes after which it was preserved at 4°C.

### Procedure 9: Tests to Verify Cell Quality

In order to verify the quality of the cells, an endotoxin test, sterility test, and analysis of cell surface antigens were carried out the day before the cells were to be prepared for final administration after the eight to sixteen-day cultivation period. For verifying, approximately 3ml of the cultivation fluid was collected, as a test sample, with a 5ml syringe having a 38mm by 18-gauge needle (Terumo Corporation). For the endotoxin test, 1 ml of the sample was dispensed in a 5ml round bottom test tube (Becton, Dickinson and Company, No.352054) and the test tube capped. Five-hundred microliters (500µl) of the sample were spread on a chocolate agar plate (Nikken Bio Medical Laboratory Inc.) for the sterility test. Five-hundred microliters (500µl) of the sample were dispensed to each of three 5ml round bottom test tubes for the cell surface antigen analysis.

### Procedure 10: Endotoxin Test

The test sample collected in Procedure 9 was centrifuged at 24°C at 1800 rpm for 5 minutes. Before the test, 80µl of pure water and 20µl of the cultivated supernatant were mixed into a main reagent solution (Toxi Color LS-50S set, Seikagaku Corporation, No.020130) prepared in a round bottom 5ml test tube after which the solution was incubated in a water bath at 37°C for 35 minutes. Concurrently, 100µl of pure water and an endotoxin standard solution (CSE-L set, Seikagaku Corp., No.020055), were mixed with the main reagent solution respectively and incubated in the water bath together with the sample for use as a blank and positive control.

The reaction was terminated by the addition of 400µl of 0.8mol/l acetic acid solution after which 380µl of the resulting solution was transferred to a 96-well microplate. Absorbency reference wavelengths were measured at 405nm and 655nm using a microplate reader. As a result of the standard solution being prepared to approximately 0.1 EU/ml, a multiple of five of the test sample measurement value was within a result of the standard, and the test sample was thus determined to meet the standard. All results were determined to have met this standard.

### Procedure 11: Sterility test

The sample prepared in Procedure 9 was seeded on a chocolate agar plate and stored in an incubator at 37 °C. The sterility determination was made the next day, that is, before preparation of the final cell formulation which would be used for treatment. As there were no colonies of microorganisms existing on the plate, a negative determination was made, and thus rendered all results negative.

### Procedure 12: Cell Surface Antigen Analysis Prior to Formulating Cells for Administration

Cell surface antigen analysis was conducted using the same method described in Procedure 5. The results are shown in Table 2.

**Table 2: Cell Surface Antigen Analysis After Cultivation**

| | Healthy Canine 1 | | Healthy Canine 2 | |
|---|---|---|---|---|
| | 1 | 2 | 1 | 2 |
| CD3 positive T cell | 96.0% | 92.0% | 79.6% | 96.6% |
| B cell positive cell | 2.5% | 2.3% | 1.4% | 0.9% |
| CD4 positive cell | 4.6% | 10.2% | 11.4% | 12.5% |
| CD8 positive cell | 94.7% | 90.0% | 92.0% | 91.8% |

### Procedure 13: Preparing the Cell Formulation

The formulation was prepared for administration on the day after it was determined that the cells met the endotoxin, sterility, and cell surface antigen analysis standards within a period eight to twelve days from initiation of cultivation. Preparation was as follows. The entire 250ml quantity of the incubation broth in the flask was transferred, during cultivation, to a centrifuge tube and centrifugally separated at 24°C at 1,000 rpm for 8 minutes. The resulting supernatant was discarded by decanting and the cells dispersed using a vortex mixer. Fifty milliliters (50ml) of the physiological saline solution containing 0.5% of autologous serum (as prepared in Procedure 8) was then added and mixed into the solution.

The resulting solution was separated in a centrifuge again at 24°C and 1,700 rpm for 8 minutes, the supernatant discarded, and the cells dispersed using the vortex mixer. In order to assure adequate cell suspension, 10ml of the physiological saline solution containing 2% autologous serum were added to the cell dispersion solution for treatment of canines weighing less than 10kg, and 30ml was added for canines weighing more than 10kg. The cell suspensions were filtered through a cell strainer (Beckton, Dickinson & Co., No. 352360) after which 10µl of the cells were suspended in 1ml of physiological saline solution with 2% albumin in preparation for measuring the number of cells.

### Procedure 14: Counting the Number of Cells After Formulation

Ten microliters (10µl) of the final formulation prepared in Procedure 13 were sampled and the number of cells measured using the same method described in Procedure 4. The 10µl of the sample was mixed with 20µl trypan blue staining solution (Sigma Corp., No.T8154), 10µl of the resulting solution was placed in an improved Neubeier Hematocytometer, and then the stained cells were counted under a microscope to determine the survival rate. It was determined that the cell volume was 3.3×10⁹ and 2.2×10⁹ for the healthy canine subject 1, and 4.1×10⁹ and 3.0×10⁹ for the healthy canine subject 2. All cell survival rates were greater than 95% and were thus considered satisfactory.

### Procedure 15: Administering the Activated Lymphocytes

The autologous cell suspension prepared in Procedure 13 was administered to the healthy canine subjects 1 and 2. No adverse effects were observed other than a slight rise in body temperature..

### [Second Embodiment]

One to 12 treatments of the activated lymphocytes, which were formulated in the same manner as described in the first embodiment, were administered to the nine tumorous canines. Quality-of-life improvements were noted in the form of increased appetite, improvement in appearance of coat, and the ability of the animals to walk approximately twice as far as before treatment. The results of the administrations are shown in Table 3.

**Table 3: Results of treating tumorous canines with lymphocytes cultivated at 37°C**

| Subject | Canine Type | Weight | Sex | Age | Cancer Type | Number of administered cells | No.of treatments | Quality of life improvement |
|---|---|---|---|---|---|---|---|---|
| 1 | Labrador Retriever | 30Kg | F | 6 years 11 months | chronic granulomatuous hepatitis | 19×10⁸~ 5.0×10⁸ | 12 | vigor restore, improvement in fur appearance |
| 2 | Bernese Mountain Dog | 39Kg | F | 9 years | adenocarcinoma | 0.7×10⁸~ 4.5×10⁸ | 9 | vigor restore, recovery of appetite |
| 3 | Shetland Sheepdog | 10.7Kg | M | 8 years 8 years 3 moths 3.0×10⁸ | rectal gland cancer | 1.1×10⁸~ 1.1 × 10⁸~ | 5 | vigor restore, recovery of appetite |
| 4 | Italian Greyhound | 8 Kg | M | 6 years 11 months | cervical fiber cancer | 1.2 × 10⁸~ 7.6 × 10⁸ | 4 | increased energy |
| 5 | Shetland Sheepdog | 9.1Kg | F | 14 years 2 months | soft tissue sarcoma | 1.0×10⁸~ 3.1 × 10⁸ | 5 | increased vigor, improved health, disappearance of tumor |
| 6 | Flat Coated Retriever | 29.5Kg | F | 7 years 11 months | blood vessel sarcoma | 1.7 × 10⁸~ 3.9 × 10⁸ | 5 | excellent recovery from surgery, normal vigor |
| 7 | Golden Retriever | 35.6Kg | F | 12 years 10 months | blood vessel sarcoma | 0.7 × 10⁸ ~ 3.9 × 10⁸ | 5 | Maintained healthy fur and vigor |
| 8 | Golden Retriever | 32.7Kg | M | 10 years 5 months | rectal gland cancer | 0.7 × 10⁸ 4.7 × 10⁸ | 4 | no change |
| 9 | Cairn Terrier | 7.5Kg | F | 9 years 11 months | mammary gland malignant mixed tumor | 4.7 × 10⁸ | 1 | improvement in appearance of fur, vigor restore |

### [Third Embodiment]

Peripheral blood lymphocytes taken from the healthy canine subjects 3 and 4 were separated by the procedure described in the first embodiment. The canine lymphocytes were cultivated through a method essentially similar to that described in the first embodiment, but at cultivation temperatures of 37°C (±0.2°C), 38°C (±0.2°C) and 39°C (±0.2°C). The results for lymphocyte volumes which were grown fifteen to twenty-fold are shown in Table 4 (for the healthy canine subject 3), in Table 5 (for the healthy canine subject 4), in Table 6, and in the Fig. 1 graph which shows a comparison of cell growth at different cultivation temperatures. Among the three cultivation temperatures of 37°C, 38°C, and 39°C, optimum cell grown was achieved for the cells cultivated at 39°C.

**Table 4: Cell quantity and surface antigens at different cultivation temperatures (healthy canine subject 3)**

| | Days cultivated | Cell Quantity | Survival Rate | CD3 | B Cell | CD4 | CD8 |
|---|---|---|---|---|---|---|---|
| cultivation temp. | 0 | 5.00E+06 | - | 73.51% | 24.67% | 41.72% | 26.14% |
| 37°C | 9 | 7.50E+07 | 84.5% | 78.5% | 5.99% | 46.08% | 69.68% |
| 38°C | 7 | 1.05E+08 | 93.7% | 94.26% | 6.74% | 23.76% | 83.32% |
| 39°C | 6 | 8.00E+08 | 89.0% | 99.32% | 6.43% | 31.38% | 78.17% |

**Table 5: Cell quantities and surface antigens at different culture temperatures (healthy canine subject 4)**

| | Days Cultivated | Cell Quantity | Survival Rate | CD3 (%) | B Cell (%) | CD4 (%) | CD8 (%) |
|---|---|---|---|---|---|---|---|
| cultivation temp. | 0 | 5.00E+06 | - | 81.28 | 27.17 | 44.68 | 24.53 |
| 37°C | 7 | 9.00E+07 | 92.2% | 94.24 | 5.57 | 27.54 | 78.58 |
| 38°C | 7 | 1.05E+08 | 92.8% | 95.83 | 4.96 | 23.62 | 82.26 |
| 39°C | 6 | 1.00E+08 | 89.0% | 96.96 | 7.04 | 27.08 | 80.09 |

**Table 6: Days required to increase cell quantity by a factor of ten**

| Cultivation Temperature | Sample | Days | Average (days) |
|---|---|---|---|
| 37°C | Sample 3 | 8.4 | 7.3 |
| | Sample 4 | 6.2 | |
| 38°C | Sample 3 | 6.2 | 6.2 |
| | Sample 4 | 6.2 | |
| 39°C | Sample 3 | 5.4 | 5.3 |
| | Sample 4 | 5.2 | |

As shown in Table 6, the cultivation period required to grow the number of cells ten-fold averaged 7.3 days at 37°C, 6.2 days at 38°C, and 5.3 days at 39°C. It was determined that the time required for cultivation could be shortened by a few days.

### [Fourth Embodiment]

In the fourth embodiment, cultivation was carried out under the same conditions as those described in the first embodiment with the exception that the temperature was increased between 38°C and 39°C in an environment having a CO₂ concentration of 5.0 (±0.2%) and 95.0% (±5.0%) humidity in a large air jacket-type CO₂ incubator. The obtained activated lymphocytes were administered one and two times to five tumorous canines. Quality-of-life improvements were observed in the form of increased appetite, improvements in coat quality, and the ability of the canines to walk approximately twice as far as before treatment. The results are shown in Table 7.

**Table 7: Results obtained from administering of lymphocytes, cultivated at a temperature over 38°C, to tumorous canines.**

| Canine No. | Canine Type | Weight | Sex | Age | Cancer Type | Number of administered cells | Number of treatments | Improvement of QOL |
|---|---|---|---|---|---|---|---|---|
| 1 | Labrador Retriever | 30kg | F | 6 years 11 months | chronic granulomatuous hepatitis | 3.7 × 10⁸ | 12 | recovery of vigor, improvement in quality of coat |
| 5 | Shetland Sheepdog | 9.1kg | F | 14 years 2 months | soft tissue sarcoma | 1.9 × 10⁸ | 5 | increased vigor, condition maintained (disappearance of tumor) |
| 7 | Golden Retriever | 35.6kg | F | 12 years 10 months | blood vessel sarcoma | 1.9 × 10⁸ | 5 | Maintained coat quality and vigor |
| 9 | Cairn Terrier | 7.5kg | F | 9 years 11 months | mammary gland malignant mixed tumor | 1.1 × 10⁸ | 1 | improvement in coat quality, recovery of vigor |
| 10 | Labrador Retriever | 31kg | M | 1 year 5 months | rhabdomyosarcoma of bladder | 2.8 × 10⁸~ 4.7 × 10⁸ | 2 | vigor maintained |

While the no.5 tumorous canine in Tables 3 and 7 died after six administrations of the activated lymphocytes, the death was unrelated to its tumorous condition. It was confirmed by autopsy, however, that a tumor, which could not be surgically removed, had disappeared.

### [Fifth Embodiment]

In the fifth embodiment, the lymphocytes were cultivated under conditions essentially the same as those described in the third embodiment that the temperature was raised between 38°C and 39°C in the large air jacket-type CO₂ incubator in which the environment had CO₂ concentration of 5% (±0.2%) and 95% (±5.0%) humidity. A portion of the cells were then cryogenically preserved, thawed, incubated, and then administered to the test subjects. Methods essentially similar to those described in Procedures 1 through 6 were applied before the commencement of cultivation.

### Procedure'16: Cryogenic Preservation of a Portion of the Cells

Three to five days following the cultivation period during which the cells were activated and propagated, 10 to 30ml of the cultivating medium was added to the same flask until the volume in the flask reached 50ml. The number of cells was then counted during the period four to seven days after cultivation commenced. The cells were cryogenically preserved when their number exceeded 1.0×10⁶/ml.
With the cells still under cultivation, 30 to 50ml of the cultivating solution was transferred to each of 50ml test tubes and then separated in a centrifuge at 24°C and 1,200 rpm for 5 minutes. Their supernatants were discarded by decanting after which 1.8ml of a cell cryopreservation medium (Bambanker^{™}, Lymphotec Inc.) was added to each of the tubes then the solutions were thoroughly mixed with a vortex mixer. One point eight milliliters (1.8ml) of the cell suspension was added to each of 2ml cryotubes (Corning Incorporated, No.430289), the tubes placed in a Bicell unit (a cryopreservation container manufactured by Nihon Freezer Co., Ltd.), and stored in a deep freezer (Sanyo Electric Co., Ltd. No.MDF-192) at a temperature of -85°C. One day later they were moved to a liquid nitrogen tank (My Sciences Co., Ltd.) for preservation of biological testing samples.

### Procedure 17: Thawing and Cultivating the Cryogenically Preserved Cells

The cryogenically preserved cells prepared in Procedure 16 were thawed and cultivated as follows. The frozen tubes were taken out of the liquid nitrogen tank and thawed in a heating block (Taitec Co., Ltd., Dryothermic Unit No.DTU-28) for 4 minutes. The entire contents was placed in 10ml of washing solution using a Pasteur pipette, after which the resulting cell suspension was separated in a centrifuge at 24°C at a 1,200 rpm for 3 minutes.

After the supernatant was discarded, the dispersed cell pellets were suspended in 15 to 20ml of the cultivating medium (400 ml of RPMI1640+7S, 4.5ml of 35,000IU/ml human rIL-2, and 45ml of FBS) then transferred to a 50ml test tube from which 10µl was sampled into a trypan blue staining solution for cell counting. One to two milliliters (1 to 2ml) of the remaining cell suspension were placed into each well of an untreated 24-well plate (Sumitomo Bakelite Co., Ltd., No.MS-80240) after which incubation was initiated in a large jacket-type CO₂ incubator at 37°C (±0.5°C) within an environment of 5% (±0.2%) CO₂ and 95% (±5.0%) humidity.

Cells which began cultivation in less than 20ml of the cultivating medium were examined with a microscope one to three days following their activation and propagation after which 1 to 2ml of the cultivating medium was placed in each well. The cell counting solution with trypan blue staining solution was mixed sufficiently after which 10µl of the solution was placed in an improved Neubeier Hemocytometer where the number of unstained living cells and stained dead cells were ascertained under a microscope.

### Procedure 18: Activating the Cryogenically Preserved Cells

Cells cultivated according to the method described in Procedure 17 were observed with a microscope. Two to four days following the incubation period during which the cells were activated and propagated, 20 to 30ml of the still cultivating cell solution was transferred from the wells to a culture flask prepared in the same manner as described in Procedure 1 using a pipette, then was added 20 to 30ml of cultivating medium (400ml of RPMI1640+7S, 4.5ml of 35,000 IU/ml human rIL-2, and 45ml of FBS) from which cultivation continued.

### Procedure 19: Propagating and Expanding Culture of the Cryogenically Preserved Cells

The cells cultivated in Procedure 18 were observed through a microscope. The cells adhering to the bottom of the flask were removed by tapping the flask two to four days after cultivation during which the cells were activated and propagated. All of the cells were then transferred to a 225cm² culture flask (Corning Costar Company: No.3000), an additional 50ml of the cultivating medium (400ml of RPMI1640+7S, 4.5ml of 35,000IU/ml human rIL-2, and 45ml of FBS) was added, and cultivation was allowed to continue.

In order to prepare the formulation for administration to the canine test subjects, the cells were observed through a microscope six to eight days from the start of cultivation during which the cells were activated and propagated, after which 150ml of the cultivating medium (400ml of RPMI1640+7S, 2.25ml of 35,000IU/ml human rIL-2, and 45ml of FBS) were added.

### Procedure 20: Testing the Cryogenically Preserved Cells

The cultivated and cryogenically preserved cells were tested in the same manner as described in Procedures 9 through to 12.

### Procedure 21: Preparation and Counting the Cryogenically Preserved Cells for Therapeutic Administration

The cryogenically preserved cells, which were cultivated as described in Procedures 13 and 14, were counted and prepared for final administration.

### Procedure 22: Administering the Cryogenically Preserved Cells

When the autologous cell suspension prepared in Procedure 13 was administered only once to the canine patient, a clear improved coat appearance, and the ability of the animal to walk a distance approximately twice as far as previously. The results are shown in Table 8.

**Table 8: Results of tratment in which cryogenically frzen lymphocytes were thawed, reactivated, and administered to a canine patient.**

| | | | | | | | | |
|---|---|---|---|---|---|---|---|---|
| Patient No. | Canine type | Weight | Sex | Age | Cancer Type | Quantity of administered cells | No. of treatments | Improvement quality-of-life |
| 9 | Cairn Terrier | 7.5Kg | F | 9 years 11 months | malignant mammary gland tymor | 4.7 X 10⁸ | 1 | improvemment in coat quality recovery of vigor |

### Figure 1: Comparison of Cell Propagation by Temperature

Comparison of differences in canine lymphocyte propagation according to cultivation temperature
healthy canine subject 3
healthy canine subject 4
Total Cell Count
Number of Days Under Cultivation

## Claims

1. Use of canine peripheral blood lymphocytes propagated and activated in the presence of a combination of solid-phase anti-canine CD3 antibodies and interleukin-2 (IL-2) in the manufacture of a medicament for treating a tumorous canine.

2. Use according to Claim 1 wherein said interleukin-2 (IL-2) is a human interleukin-2 (human IL-2).

3. Use according to Claim 1 wherein the temperature under which the canine peripheral blood lymphocytes are propagated and activated lies within a range of from 37°C to 41 °C.

4. Use according to Claim 1 wherein the temperature under which the canine peripheral blood lymphocytes are propagated and activated lies within a range of from 38°C to 40°C.

5. A pharmaceutical formulation for treatment of a tumorous canine wherein said formulation comprises canine peripheral blood lymphocytes which have been propagated and activated in the presence of a combination of solid-phase anti-canine CD3 antibodies and interleukin-2 (IL-2).

6. The pharmaceutical formulation for treating a tumorous canine according to Claim 5, wherein the interleukin-2 (IL-2) is a human interleukin-2 (human IL-2).

7. The pharmaceutical formulation for treating a tumorous canine according to Claim 5, wherein the temperature under which the canine peripheral blood lymphocytes are propagated and activated lies within in a range of from 37°C to 41 °C.

8. The pharmaceutical formulation for treating a tumorous canine according to Claim 5, wherein the temperature under which the canine peripheral blood lymphocytes are propagated and activated lies within in a range of from 38°C to 40°C.

9. A method for cryogenically preserving cells for treating a tumorous canine wherein canine peripheral blood lymphocytes are propagated and activated in vitro in the presence of a combination of solid-phase anti-canine CD3 antibodies and interleukin-2 (IL-2) after which the lymphocytes are cryogenically preserved, thawed and restored at use, and reactivated for preparing a pharmaceutical formulation.

10. The method for cryogenically preserving cells for treating a tumorous canine according to Claim 9 wherein the interleukin-2 (IL-2) is a human interleukin-2 (human IL-2).

11. The method for cryogenically preserving cells for treating a tumorous canine according to Claim 9 wherein the temperature under which the canine peripheral blood lymphocytes are propagated and activated in vitro lies within a range of from 37°C to 41 °C.

12. The method for cryogenically preserving cells for treating a tumorous canine according to Claim 9 wherein the temperature under which the canine peripheral blood lymphocytes are propagated and activated in vitro lies within in a range of from 38°C to 40°C.
